(19) Europäisches Patentamt
European Patent Office
Office européen des brevets

(11) Publication number: 0 366 797 A1

(12) EUROPEAN PATENT APPLICATION
published in accordance with Art. 158(3) EPC

(21) Application number: 89901299.1

(22) Date of filing: 10.01.89

(86) International application number: PCT/JP89/00020

(87) International publication number: WO 89/10341 (02.11.89 89/26)

(51) Int. Cl.[5] C07B 39/00, C07C 17/08, C07C 17/20, C07C 19/08

(30) Priority: 28.04.88 JP 104305/88
28.04.88 JP 104306/88

(43) Date of publication of application: 09.05.90 Bulletin 90/19

(84) Designated Contracting States: DE FR GB IT NL

(71) Applicant: SHOWA DENKO KABUSHIKI KAISHA
13-9, Shiba Daimon 1-chome
Minato-ku, Tokyo 105(JP)

(72) Inventor: HIRAYAMA, Shuji Showa Denko Kabushiki K.K.K.
5-1, Ogi machi Kawasaki-ku
Kawasaki-shi Kanagawa 210(JP)
Inventor: KOBAYASHI, Hiroshi Showa Denko Kabushiki K.K.K.
5-1, Ogi machi Kawasaki-ku
Kawasaki-shi Kanagawa 210(JP)
Inventor: ONO, Hiromoto Showa Denko Kabushiki K.K.K.
5-1, Ogi machi Kawasaki-ku
Kawasaki-shi Kanagawa 210(JP)
Inventor: TOMODA, Seiichi Showa Denko Kabushiki K.K.K.
5-1, Ogi machi Kawasaki-ku
Kawasaki-shi Kanagawa 210(JP)
Inventor: TAKAICHI, Tsuyoshi Showa Denko Kabushiki K.K.K.
5-1, Ogi machi Kawasaki-ku
Kawasaki-shi Kanagawa 210(JP)

(74) Representative: Redies, Bernd, Dr. rer. nat.
COHAUSZ & FLORACK Patentanwaltsbüro
Schumannstrasse 97 Postfach 14 01 20
D-4000 Düsseldorf 1(DE)

# (54) PROCESS FOR PRODUCING ORGANOFLUORINE COMPOUND.

(57) A process for producing an organofluorine compound, which comprises reacting an organochlorine



Xerox Copy Centre

compound or an organic unsaturated compound with hydrogen flouride in the presence of a catalyst comprising a support of active alumina having fine pores 70 to 90 % of which have an average pore size of 40 to 500 Å carrying thereon a fluoride of at least one metal selected from among nickel, cobalt, iron, manganese, chromium, copper and silver.

TITLE M(
see front

# PROCESS FOR PREPARATION OF ORGANIC FLUORINE COMPOUND

## TECHNICAL FIELD

The present invention relates to a process for the preparation of an organic fluorine compound. The organic fluorine compound prepared according to the process of the present invention can be used as a cooling medium, an aerosol propellant, a foaming agent, a detergent and the like.

## BACKGROUND ART

Various processes have been proposed as the means for preparing an organic fluorine compound by reacting an organic chlorine compound with hydrogen fluoride. The conventional processes are roughly divided into the liquid phase process and the gas phase process. In the past, the former process was mainly investigated, but recently the latter process has been mainly studied. The selection of a catalyst is especially important in the production of an organic fluorine compound by the gas phase process.

For example, in case of the synthesis of 1-chloro-2,2,2-trifluoroethane or 1,2,2,2-tetrafluoroethane, it is known that a chromium (III) compound such as chromium (III) chloride or chromium (III) fluoride, or chromium oxyfluoride obtained by reacting chromium fluoride with oxygen or by reacting chromium oxide or chromium hydroxide with hydrogen fluoride is used for the synthesis of the former compound by the gas phase fluorination of trichloroethylene, and Japanese Examined Patent Publication No. 43-10601, Japanese Unexamined Patent Publication No. 53-105404 and Japanese Unexamined Patent Publication No. 56-24050 teach that a chromium type catalyst is used for the synthesis of the latter compound by the fluorination of 1-chloro-2,2,2-trifluoroethane. Catalysts other than the chromium type

catalysts are known as catalysts for the synthesis of organic fluorine compounds by the gas phase reaction, but as apparent from the above-mentioned examples, in practical chromium type catalysts are mainly used.

Nevertheless, the known chromium type catalysts are defective in that the industrial preparation is difficult and the catalyst stability is poor, and further improvements of the conversion and productivity are desired. It is therefore an object of the present invention to provide a process for preparing an organic fluorine compound by the gas phase reaction using a fluorination catalyst showing an excellent conversion and productivity, in which the above-mentioned problems are overcome.

DISCLOSURE OF THE INVENTION

The object of the present invention can be attained by using a catalyst comprising active alumina having a uniform fine pore distribution as a catalyst carrier and a fluoride of a metal selected from the group consisting of cobalt, nickel, iron, manganese, chromium, copper and silver as a catalyst component, which is supported on the active alumina.

More specifically, in accordance with the present invention, there is provided a process for the preparation of an organic fluorine compound, which comprises reacting an organic chlorine compound or an organic unsaturated compound with hydrogen fluoride in the presence of a catalyst comprising a catalyst carrier composed of active alumina in which pores having an average pore size of 40 to 500 Å occupy 70 to 90% of the whole pores, and at least one fluoride of a metal selected from the group consisting of nickel, cobalt, iron, manganese, chromium, copper and silver, which is supported on the catalyst carrier.

BEST MODE OF CARRYING OUT THE INVENTION

The active alumina valuable as the carrier of the catalyst used in the process of the present invention

will now be described in detail.

The active alumina is hydrous alumina, and according to the X-ray diffractometry, the active alumina is identified as intermediate alumina such as κ-, θ-, δ, τ-, η-, X-, or ρ-alumina, in which a non-crystalline (amorphous) portion is predominant and crystallization is not substantially advanced, for example, boehmite, pseudoboehmite or a calcination product thereof. Usually, τ- or η-alumina is recommended as the active alumina. When both of τ- and η-phases are present in alumina, these phases cannot be distinguished from each other by the X-ray diffractometry, and this alumina is called "τ/η-alumina".

In general, the active alumina is prepared by thermal decomposition of hydrated alumina, that is, by subjecting hydrated alumina to controlled heating for removing the majority of water. As the starting material for hydrated alumina, there can be mentioned alumina trihydrate prepared by the Bayer process, an aluminum salt, an alkali metal aluminate, an aluminum alkoxide and metallic aluminum. As the hydrate, there can be mentioned gibbsite, bialite and nordstrandite [they have a chemical formula of $Al(OH)_3$], and boehmite and diaspore (they have a chemical formula of $Al_2O_3 \cdot H_2O$ or AlOOH).

Excepting where hydrated alumina by the Bayer process is used, an alumina gel is formed. At this step, various alumina hydrates are formed singly or in the form of a mixture according to the conditions such as the temperature, pH, time, and starting material concentration. These hydrates are decomposed into different alumina phases and products differing in pore structure, surface area and physical properties are obtained.

As the process for the preparation of active alumina, industrially worked are (A) a process in which

hydrated alumina such as gibbsite or boehmite is heated and dehydrated, (B) a process in which hydrated alumina (gel) obtained by reaction between an aqueous solution of sodium aluminate and an aqueous solution of aluminum sulfate or carbon dioxide gas or by reaction between powdery sodium aluminate and sulfurous acid gas is dehydrated, (C) a process in which hydrated alumina obtained by adding ammonia or an aqueous solution of a hydroxide or carbonate of an alkali metal to an aqueous solution of an aluminum salt (such as aluminum sulfate, aluminum nitrate or aluminum chloride) is dehydrated, hydrated alumina obtained by the homogeneous precipitation method comprising adding urea to an aluminum salt and heating the mixture is dehydrated or alumina hydrogen obtained from basic aluminum sulfate (a kind of alumina hydrosol) formed by adding a carbonate to aluminum sulfate is dehydrated, (D) a process in which an aluminum salt is heated and decomposed, and (E) a process in which aluminum iso-propoxide is hydrolyzed.

Of these active alumina products, active alumina synthesized from alumina trihydrate by the Bayer process is most widely available. This alumina is formed by heat-treating and activating obtained gibbsite at about 400°C in an air current, and this alumina is X-ray-diffractometrically $\tau/\eta$-alumina containing a small amount of boehmite. The specific surface area is about 250 $m^2/g$, and the alumina has a broad pore size distribution of from 35 Å to 10 $\mu$ and many fine pores having a pore size smaller than 35 Å are present. The pores include cylindrical and spherical pores and the like. In general, the pore size distribution is determined by an Hg-porosimeter of 50000 psi (pore sizes of 35 Å to 177 $\mu$ are measured). The composition of this alumina comprises about 0.9% of $Na_2O$ and several ppm of $SiO_2$ and $Fe_2O_3$ and it is difficult to reduce the $Na_2O$ content below 0.05%.

Active alumina also can be prepared by rapidly

activating hydrated alumina by the Bayer process at a temperature of 400 to 800°C. Since the formation of boehmite and formation of a decomposition product are strictly controlled in this process, the pattern of $\tau/\eta$-alumina is weak and an alumina which is almost amorphous is obtained. By an agglomeration or rehydration of this alumina, a product having a spherical form or other form is obtained. In this active alumina, the crystallite size and pore size are smaller than in the above-mentioned active alumina. The specific surface area is 300 to 350 $m^2/g$.

Where alumina gel is used as the starting material [the above-mentioned processes (B) and (C)], after the precipitate is washed with water and completely drained, the alumina is X-ray-diffractometrically peseudo-beohmite. Industrially, this cake is dried, pulverized and extruded into a cylindrical form or spray-dried into spherical fine particles and molded into spheres or pellets. Activation is effected by a heat treatment to obtain active alumina. This active alumina is X-ray-diffractometrically $\tau/\eta$-alumina which is almost amorphous. The composition of this active alumina contains a small amount of $SiO_2$ or 2 to 3% of $SO_3$. Gels differing in structure can be synthesized according to this process, and the specific surface area is 300 to 600 $m^2/g$ and the active alumina has many fine pores.

The so-obtained active alumina has a pore volume of 0.3 to 0.8 ml/g and a specific surface area of 150 to 350 $m^2/g$ and has pores having an average pore size of about 40 to about 150 Å. The pore size distribution is wide and pores having a size of 40 to 500 Å are distributed only in an amount of about 20%.

In the active alumina used for the catalyst of the present invention, preferably the ratio of pores having a pore size of 40 to 500 Å is concentrated to the range of 70 to 90%. Active alumina in which pores having a pore size smaller than 40 Å are predominantly

distributed are difficult to obtain industrially, and when this active alumina is used as the catalyst carrier, the activity is improved but the selectivity is poor and the catalyst life is too short. Active alumina in which the pore size distribution is concentrated to 500 Å is not preferred because a sufficient catalyst activity is not obtained and the yield is reduced.

The pore volume of active alumina is determined by the specific surface area and the pore size distribution, and if the pore volume is smaller than 0.55 ml/g, an appropriate specific surface area and pore structure cannot be obtained, and often the catalyst activity is low and the yield is insufficient. Accordingly, in the active alumina used in the present invention, preferably the volume of pores having a size of at least 40 Å is at least 0.55 ml/g, especially 0.55 to 1.6 ml/g.

As an example of the process for the industrial production of active alumina preferably used in the present invention, there can be mentioned the sol-gel oil-dropping process [resembling the above-mentioned process (C)]. According to this process, the chemical properties of the spherical sol can be adjusted, or the bulk density, specific surface area, pore volume, pore size, and pore size distribution of the formed active alumina can be optionally adjusted by changing the chemical operation of sol-gel.

As the preferred active alumina, there can be mentioned a product obtained by decomposing a trialkylaluminum to aluminum monohydrate [process resembling the above-mentioned process (E)] and calcining this hydrated alumina to $\tau$-alumina. When this alumina is pressed or extruded into a pellet, a product can be obtained having a higher purity, a more uniform particle, and a better pore size distribution than naturally produced alumina.

The foregoing alumina to be used as the catalyst

carrier is commercially available, and active alumina containing 70 to 90% of pores having an average pore size of 40 to 500 Å is selected from among commercially available products. In view of the handling ease at the time of charging or discharging the catalyst into or from the reactor, this active alumina is preferably shaped into particles, beads or extrusion-molded bodies having a diameter of less than 20 mm, especially several mm.

In connection with impurities in the active alumina, the sodium content must be lower than 100 ppm, preferably as low as possible. Active alumina having a silicon content lower than 300 ppm and an iron content lower than 100 ppm is preferably selected. If sodium, silicon and iron are present in the form of oxides in the carrier, they exert an activity-inhibiting action and a reaction-inhibiting action such as promotion of formation of silicon fluoride by fluoric anhydride, isomerization or disproportionation. Accordingly, preferably these impurities are not present in the catalyst.

As the metal constituting the catalyst component in the present invention, there can be mentioned nickel, cobalt, iron, manganese, chromium, copper and silver. At the metal source, there are preferably used inorganic and organic salts of these metals. For example, in the case of nickel, there can be mentioned nickel chloride, nickel nitrate, nickel hydroxide, nickel sulfate, nickel carbonate, basic nickel carbonate, nickel formate, nickel oxalate, nickel acetate, nickel oxide, nickel sesquioxide, nickel dioxide, hydrous nickel sesquioxide, nickelous fluoride, nickelic fluoride, hydrous nickelous fluoride, nickel oxyfluoride and nickel fluoride. Metallic nickel can be used if desired. Of these nickel salts, water-soluble salts such as nickel fluoride, nickel nitrate and nickel sulfate are preferably used. Similar salts and compounds of cobalt, iron, manganese,

chromium, copper and silver can be used, as long as they are practically available. In connection with these metals as well as nickel, water-soluble salts such as chlorides, nitrates and sulfates are preferably used.

Known various processes can be applied to the production of the catalyst. For example, there can be adopted a process in which active alumina is impregnated with an aqueous solution of a metal salt, dried calcined and subjected to the hydrofluorination treatment, or the metal salt is once reduced to a metal with hydrogen and the metal-deposited active alumina is subjected to the hydrofluorination treatment. Namely, preferably, after deposition of the metal or metal compound on the active alumina, the deposited metal or metal compound is reacted with hydrogen fluoride to convert at least a part of the metal to a fluoride (the hydrofluorination treatment). Where a metal fluoride is deposited on the carrier, the treatment with hydrogen fluoride is sometimes unnecessary.

In the production of the catalyst, preferably the active alumina is impregnated with an aqueous solution of a metal salt or an aqueous solution of a metal salt is absorbed in the active alumina in an amount corresponding to the maximum absorbable amount of water, and then, the active alumina is hot-air-dried and calcined in an air current.

Drying is preferably carried out at a temperature of 50 to 120°C, and calcination is preferably carried out in a circulation of an oxygen-containing inert gas, especially air, that is, by passing air at [SV]o (the space velocity as calculated at 0°C under atmospheric pressure) of 100 to 1000 $hr^{-1}$ and a temperature of 200 to 500°C. Where the metal-deposited active alumina is dried and calcined in an air current, an abrupt generation of heat occurs at a temperature higher than 150°C. Accordingly, preferably the temperature is maintained at a level lower than 500°C by controlling

the temperature. If the calcination temperature is lower than 200°C, the catalytic activity is low, and if the calcination temperature is higher than 500°C, the effective surface area of the catalyst is reduced (sintering occurs), and therefore, the catalyst activity is reduced. The metal content in the catalyst can be optionally changed, but in view of the activity and the limit of the deposition amount preferably the metal content in the catalyst is 5 to 30% by weight.

Prior to the hydrofluorination treatment, the metal oxide/alumina catalyst must be treated with hydrofluoric anhydride (hydrogen fluoride: HF) at a hydrofluoric anhydride concentration of 10 to 100% (at a concentration lower than 100%, hydrofluoric anhydride is diluted with an inert gas and is then used), a space velocity [SV]o of 50 to 600 $hr^{-1}$ and a temperature of 200 to 450°C to activate the metal oxide and alumina by partial fluorination. Since a generation of heat occurs during this partial hydrofluorination treatment, preferably the temperature control is carried out so that the catalyst temperature is lower than 450°C.

The reaction between the organic chlorine compound and hydrogen fluoride includes the following reactions. The basic reactions of the organic chlorine compound comprising main atomic groups $CH_2Cl$, CHCl, $CHCl_2$ , $CCl_2$ and $CCl_3$ with hydrogen fluoride (HF) are represented as follows:

$$CH_2Cl + HF \text{———} CH_2F + HCl$$
$$CHCl + HF \text{———} CHF + HCl$$
$$CCl + HF \text{———} CF + HCl$$
$$CHCl_2 + HF \text{———} CHF_2 + CHClF + HCl$$
$$CCl_2 + HF \text{———} CF_2 + CClF + HCl$$
$$CCl_3 + HF \text{———} CF_3 + CClF_2 + CCl_2F + HCl$$

The reaction between an organic chlorine compound having atomic groups CHClF, CClF, $CClF_2$ and $CCl_2F$, that is, a most intermediate fluorinated compound, and hydrogen fluoride includes the following reactions:

$$CHClF + HF \longrightarrow CHF_2 + HCl$$
$$CClF + HF \longrightarrow CF_2 + HCl$$
$$CClF_2 + HF \longrightarrow CF_3 + HCl$$
$$CCl_2F + HF \longrightarrow CF_3 + CClF_2 + HCl$$

Of course, these reactions are included in the reaction of the present invention.

The organic chlorine compound referred to in the present invention is a polyhalogenated organic compound having such organic groups as $CH_2Cl$, CHCl, CCl, $CHCl_2$ , $CCl_2$ , $CCl_3$ , CHClF, CClF, $CClF_2$ and $CCl_2F$. The organic chlorine compound may partially contain such atomic groups as $CH_2F$, CHF, CF, $CHF_2$ , $CF_2$ and $CF_3$.

Typical examples of the organic chlorine compound include carbon tetrachloride, chloroform, methylene chloride, hexachloroethane, pentachloroethane, tetrachloroethane, trichloroethane, octachloropropane, heptachloropropane, hexachloropropane, pentachloropropane, tetrachloropropane, trichloropropane, tetrachloroethylene, trichloroethylene, hexachloropropene, pentachloropropene, tetrachloropropene, trichloropropene, hexachlorobutadiene, hexachlorobetene, pentachlorobutene, tetrachlorobutene, pentachlorobutane, tetrachlorobutane, benzotrichloride, chlorinated benzotrichloride, nitrated benzotrichloride, trichloromethylated benzotrichloride, trichloromethyl group-containing ether, trichloromethyl group-containing ketone, trichloromethyl group-containing ester, trichloromethyl group-containing acid halide, trichloromethyl group-containing pyridine, trichloromethyl group-containing nitrile and polyhalogenated organic compounds in which parts of chlorine atoms of the foregoing compounds are substituted with fluorine atoms.

Among these compounds, polychlorination products of lower hydrocarbons, hexachloroacetone, benzotrichloride and derivatives thereof are industrially important.

The organic fluorine compound obtained according to

the process of the present invention is a substance formed by substituting a part or all of chlorine atoms of an organic chlorine compound as mentioned above with fluorine atoms, and little increase or decrease of the carbon number of the basic skeleton, as observed by fluorination with elementary fluorine gas, occurs. According to the process of the present invention, in principle, the basic structure of the predetermined compound is maintained throughout the reaction and changes of the molecular weight and properties by substitution of chlorine atoms with fluorine atoms are mainly observed.

The process of the present invention also can be advantageously applied to the fluorination reaction of the organic unsaturated compound. Tetrachloroethylene and trichloroethylene are included in the category of the organic unsaturated compound. For example, in the case of the reaction between trichloroethylene and hydrogen fluoride, addition reaction and substitution reaction are advanced stepwise as shown below:

$$CHCl = CCl_2 \xrightarrow{HF} CH_2Cl - CCl_2F$$

$$\xrightarrow{2HF} CH_2Cl - CF_3$$

Accordingly, the organic fluorine compound also includes a compound formed by addition of hydrogen fluoride to an organic unsaturated compound.

In the process of the present invention, the catalyst fixed bed type system and the moving bed type system can be adopted for the production of an organic fluorine compound by the gas phase fluorination of an organic chlorine compound.

The reaction temperature is generally 100 to 600°C, preferably 150 to 550°C. If the reaction temperature is too low, the reaction speed is low, and if the reaction temperature is too high, side reactions such as defluorination, disproportionation, isomerization and

coke-forming reaction occur, resulting in a reduction of the selectivity. Accordingly, too low a temperature and too high a temperature are not practically preferable.

The reaction pressure is not particularly critical, as long as the gas phase can be maintained, and a pressure of 1 to 10 atmospheres is generally adopted. In view of the operation ease and the productivity, atmospheric pressure or a pressure slightly higher or lower than atmospheric pressure is preferred. The space velocity [SV]o is 50 to 2000 $hr^{-1}$, and the amount of hydrogen fluoride is preferably 0.5 to 20 moles per mole of the organic chlorine compound. This ratio of hydrogen fluoride is important for a determination of the selectivity of the reaction, and the ratio of hydrogen fluoride is appropriately decided in view of the ratio of substitution of chlorine or fluorine of the organic chlorine compound.

The molar ratio of hydrogen fluoride to the starting organic chlorine compound and/or unsaturated compound will be referred to as "HF molar ratio" hereinafter.

Typical instances of the starting material and product in the process of the present invention, and preferred reaction conditions, will now be described.

1-Chloro-2,2,2-trifluoroethane (F-133a) is prepared by using trichloroethylene as the organic unsaturated compound and carrying out reaction at a temperature of 200 to 450°C and a hydrogen fluoride molar ratio of 3 to 10 to trichloroethylene.

If the reaction temperature is lower than 200°C, the conversion is low, and if the reaction temperature is higher than 450°C, the selectivity is greatly reduced. Accordingly, too low a temperature or too high a temperature is not preferred. The HF ratio is not lower than the stoichiometric ratio, and to control a formation of $CHCl{=}CF_2$ by dehydrofluorination of F-133a without a reduction of the reaction speed, preferably

the HF ratio is not higher than 10. If the HF molar ratio is higher than 10, the productivity of the catalyst is lowered and good results cannot be obtained.

1,2,2,2-Tetrafluoroethane (F-134a) is prepared by using 1-chloro-2,2,2-trifluoroethane as the organic chlorine compound and carrying out a reaction at a temperature of 300 to 420°C and a hydrogen fluoride molar ratio of 1 to 10 to 1-chloro-2,2,2-trifluoroethane.

If the reaction temperature is lower than 300°C, the conversion is low, and if the reaction temperature is higher than 420°C, the selectivity is drastically reduced. Accordingly, too low a temperature and too high a temperature are not preferred. The HF molar ratio is not lower than the stoichiometric ratio, and to control formation of $CHCl{=}CF_2$ by dehydrofluorination of F-133a without a reduction of the reaction speed of F-133a, preferably the HF molar ratio is not higher than 10. Since a separation of $CHCl{=}CF_2$ from 134a is very difficult, the formation of $CHCl{=}CF_2$ should be controlled as much as possible. If the HF molar ratio is higher than 10, the productivity of the catalyst is lowered and good results cannot be obtained.

1,1-Dichloro-2,2,2-trifluoroethane (F-123) is prepared by using perchloroethylene as the organic unsaturated compound and carrying out a reaction at a temperature of 200 to 400°C and a hydrogen fluoride molar ratio of 3 to 6 to perchloroethylene.

If the reaction temperature is lower than 200°C, the conversion is extremely low, and if the reaction temperature is higher than 400°C, the selectivity is greatly reduced. Accordingly, too low a temperature or too high a temperature is not preferred. The HF molar ratio is not lower than the stoichiometric ratio. If the HF molar ratio is higher than 6, 1,1,1,2-tetrafluorochloroethane (F-124) and 1,1,1,2,2-pentafluoroethane (F-125) are preferentially formed, and therefore,

the selectivity of the formation of F-123 is reduced.

1,1,1-Dichlorofluoroethane (F-141b) and/or 1,1,1-difluorochloroethane (F-142b) is prepared by using 1,1,1-trichloroethane as the organic chlorine compound and carrying out reaction at a temperature of 150 to 400°C and a hydrogen fluoride molar ratio of 1 to 4 to 1,1,1-trichloroethane. If the reaction temperature is lower than 150°C, the conversion is extremely low, and if the reaction temperature is higher than 400°C, the selectivity is greatly reduced. Accordingly, too low a temperature and too high a temperature are not preferred. The HF molar ratio is not lower than the stoichiometric ratio. If the HF molar ratio is higher than 4, 1,1,1-trifluoroethane (F-143a) is preferentially formed and the selectivity of formation of F-141b or F-142b is reduced, and good results cannot be obtained.

Difluoromethane (F-22) is prepared by using chloroform as the organic chlorine compound and carrying out a reaction at a temperature of 150 to 350°C and a hydrogen fluoride molar ratio of 0.5 to 3 to chloroform. If the reaction temperature is lower than 150°C, the conversion is extremely low, and if the reaction temperature is higher than 350°C, the selectivity is greatly reduced. Accordingly, too low a temperature and too high a temperature are not preferred. If the HF ratio is lower than 0.5, the productivity of the catalyst is reduced, and if the HF molar ratio is higher than 3, trifluoromethane (F-23) is preferentially formed and the selectivity of the formation of F-22 is reduced.

Tetrafluoromethane (F-14) is prepared by using difluorodichloromethane (F-12) and/or trifluorochloromethane (F-13) as the organic chlorine compound and carrying out a reaction at a temperature of 200 to 460°C and a hydrogen fluoride molar ratio of 0.5 to 3 to difluorodichloromethane and/or trifluorochloromethane.

If the reaction temperature is lower than 200°C, the conversion is extremely low and if the reaction

temperature is higher than 460°C, side reactions such as decomposition occur and the selectivity is greatly reduced. If the HF molar ratio is lower than 0.5, the productivity of the catalyst is reduced. If the HF molar ratio is higher than 3, the recovery and separation of HF from F-14 become complicated and the productivity per unit weight of the catalyst is low.

1,1,1,2-Tetrafluoroethane (F-124) and/or 1,1,1,2,2-pentafluoroethane (F-125) is prepared by using trifluorochloroethylene (F-1113) as the organic unsaturated compound and carrying out a reaction at a temperature of 200 to 450°C and a hydrogen fluoride molar ratio of 1 to 5 to trifluorochloroethylene. If the reaction temperature is lower than 200°C, the conversion is extremely low, and if the reaction temperature is higher than 450°C, side reactions such as decomposition occur and the selectivity is reduced. The HF molar ratio is not lower than the stoichiometric ratio. If the HF molar ratio is higher than 5, the separation of HF from F-124 or F-125 becomes complicated and the productivity per unit weight of the catalyst is reduced.

The present invention will now be described in detail with reference to the following examples and comparative examples.

Example 1

A catalyst was prepared by using spherical high-purity active alumina (NST-7 supplied by Nikki-Universal Ltd.) having a size of 1.6 mm and the properties described below. This active alumina was prepared by the sol/gel oil-dropping method, and pores having a pore size of about 80 Å occupied about 80% by volume of the total pores.

Pore volume: 0.6 ml/g

Average pore size: 80 Å

Total BET specific surface area: 250 $m^2/g$

Pack density: 0.70 g/ml

Particle size: 1.6 mm

$Al_2O_3$ content: higher than 99.93% by weight

Na content: lower than 10 ppm

Si content: lower than 150 ppm

Fe content: lower than 100 ppm

Into 132 ml of pure water was thrown 191.5 g of chromium chloride $CrCl_3 \cdot 6H_2O$, and the mixture was heated at 70 to 80°C on a hot water bath to form a solution. The solution was cooled to room temperature and 400 g of the above-mentioned active alumina was immersed in the solution to adsorb all of the catalyst solution in the alumina. The alumina wetted with the catalyst solution was dried to the solid at 90°C on a hot water bath. The catalyst was further dried at 110°C for 3 hours in an air-circulating hot air drier.

An SUS vessel was charged with the dried catalyst and air was circulated at a space velocity [SV]o of 540 $hr^{-1}$, and the catalyst was calcined under circulation of air at 200°C until the generation of heat in the catalyst layer stopped, and the temperature was elevated to 400°C and calcination was further conducted for 3 hours at this temperature. The calculated contents of $Cr_2O_3$ and $Al_2O_3$ were 12% by weight and 88% by weight, respectively. A reaction tube (formed of nickel) having an inner diameter of 25 mm and a height of 1 m was packed with 100 ml of the calcined catalyst. Prior to the hydrogen fluoride treatment of 1-chloro-2,2,2-trifluoroethane ($CH_2Cl-CF_3$ , F-133a), the catalyst was subjected to partial fluorination with hydrofluoric anhydride diluted with nitrogen and 100% hydrofluoric anhydride to activate the catalyst. The conditions for the treatment of the catalyst with hydrofluoric anhydride were as follows.

Hydrofluoric anhydride concentration: 25 to 100 mole%

Temperature: 250 to 420°C

[SV]o: 400 $hr^{-1}$

Treatment time: about 15 hours

The gas phase fluorination reaction of F-133a was carried out by using the so-obtained catalyst. The formed gas coming from the reactor was passed through an aqueous solution of an alkali to effect absorption of unreacted fluoric anhydride and formed HCl, and dehydration was carried out in a molecular sieve 3A column and metering was performed by a gas meter. The formed gas was analyzed by the gas chromatography. The analysis was carried out when the reaction became stationary after the passage of hydrofluoric anhydride and F-133a through the catalyst.

The fluorination reaction of F-133a was conducted under conditions of a reaction temperature of 330°C, a reaction pressure of 1 atmosphere, a hydrofluoric anhydride molar ratio (HF molar ratio) of 4.6 to F-133a and a space velocity [SV]o of 101 $hr^{-1}$. The conversion of F-133a was 20.3%, and the yield and selectivity of 1,2,2,2-tetrafluoroethane ($CH_2F-CF_3$ , F-134a) were 19.1% and 94.3%, respectively. The space time yield STY of F-134a was 15.6 (g/l of catalyst·hr).

Example 2

A catalyst was prepared by using high-purity active alumina having properties described below. In this active alumina, pores having a pore size of about 270 Å occupied about 80% by volume of the total pores.

Pore volume: 1.5 ml/g

Average pore size: 270 Å

Total BET specific surface area: 190 $m^2/g$

Pack density: 0.37 g/ml

Particle size: 3.2 mm

$Al_2O_3$ content: higher than 99.93% by weight

Na content: lower than 10 ppm

Si content: lower than 150 ppm

Fe content: lower than 100 ppm

The preparation of the catalyst was carried out in the same manner as described in Example 1, and in the

formed catalyst, the contents of $Cr_2O_3$ and $Al_2O_3$ were 24% by weight and 76% by weight, respectively. The synthesis of F-134A was carried out while adjusting the amount packed of the catalyst, the conditions for the hydrogen fluoride treatment of the catalyst and the conditions for the fluorination reaction of F-133a in the same manner as in Example 1. The results are shown in Table 1.

Examples 3 and 4

Using the same catalyst as used in Example 1, the fluorination reaction of F-133a was carried out in the same manner as described in Example 1 except that the reaction conditions were changed. The results and the reaction conditions are shown in Table 1.

Example 5

A catalyst comprising 12% by weight of $Cr_2O_3$ and 88% by weight of $Al_2O_3$ was prepared in the same manner as described in Example 1 except that 287.5 g of chromium nitrate $Cr(NO_3)_3 \cdot 9H_2O$ was used as the chromium starting material. The reaction tube was packed with 100 ml of the catalyst, and the hydrogen fluoride treatment of the catalyst and the fluorination reaction of F-133a were carried out under the same conditions as described in Example 2. The results are shown in Table 1.

Example 6

A catalyst was prepared in the same manner as described in Example 1 except that 39.9 g of $CrCl_3 \cdot 6H_2O$ and 31.9 g of $(NH_4)_2CrO_4$ were used as the chromium starting material. The chromium content was 9.5% by weight. A reaction tube was packed with 100 ml of the catalyst, and the fluorination treatment and the fluorination reaction of F-133a were carried out under the same conditions as described in Example 1. The results are shown in Table 1.

Example 7

Using the same catalyst as used in Example 2, the

test of the continuous production of F-134a was carried out by conducting the fluorination of F-133a in the same manner as described in Example 2 except that the reaction conditions were changed as indicated below.

i) Reaction Conditions

Reaction temperature: 330°C

Reaction pressure: one atmosphere

[SV]o: 250 $hr^{-1}$

ii) Test Results

| Reaction Passage Time (hours) | Conversion (%) of F-133a | Selectivity (%) of F-134a | Yield (%) of F-134a | STY of F-134a (g/l of catalyst·hr) |
|---|---|---|---|---|
| 25 | 17.1 | 97.0 | 16.6 | 36.4 |
| 50 | 18.4 | 96.8 | 17.8 | 39.0 |
| 100 | 17.4 | 96.5 | 16.8 | 36.8 |
| 150 | 18.6 | 97.0 | 18.0 | 39.4 |
| 300 | 18.7 | 97.0 | 18.1 | 39.6 |

Comparative Example 1

Using active alumina (KHA-24 supplied by Sumitomo Kagaku Kogyo) having properties described below, a catalyst comprising 12% by weight of $Cr_2O_3$ and 88% by weight of $Al_2O_3$ was prepared in the same manner as described in Example 1. This active alumina had only a broad pore size distribution within a pore size range of 40 to 500 Å, and the active alumina had about 20% of fine pores having a pore size smaller than 40 Å.

Pore volume: 0.53 ml/g

Total BET specific surface area: 150 $m^2$/g

Pack density: 0.72 g/ml

Particle size: 2 to 4 mm

$Al_2O_3$ content: higher than 98.4% by weight

Na content: lower than 1900 ppm

Si content: lower than 140 ppm

Fe content: lower than 210 ppm

A reaction tube was packed with 100 ml of the catalyst, and the hydrogen fluoride treatment and the fluorination of F-133a were carried out under the same conditions as described in Example 1. The results are shown in Table 1.

Comparative Example 2

Using a commercially available chromium oxide/alumina catalyst (pellet having a diameter of 5 mm and a height of 5 mm; $Cr_2O_3$ content = 12% by weight), the hydrogen fluoride treatment and the fluorination reaction of F-133a were carried out under the same conditions as described in Example 1.. The results are shown in Table 1.

Table 1

| Example No. | Reaction Conditions: reaction temperature (°C) | Reaction Conditions: reaction pressure (atmosphere) | Reaction Conditions: HF/F-133a molar ratio | Reaction Conditions: $[SV]_o$ ($hr^{-1}$) | Reaction Results: Conversion (%) of F-133a | Reaction Results: Selectivity (%) of F-134a | Reaction Results: Yield (%) of F-134a | Reaction Results: STY of F-134a (g/l of catalyst•hr) |
|---|---|---|---|---|---|---|---|---|
| Example 2 | 330 | 1 | 4.6 | 101 | 18.8 | 95.7 | 18.0 | 15 |
| Example 3 | 330 | 1 | 8.4 | 109 | 26.8 | 96.3 | 25.8 | 14 |
| Example 4 | 390 | 1 | 8.0 | 430 | 31.2 | 91.0 | 28.4 | 62 |
| Example 5 | 330 | 1 | 4.6 | 101 | 20.0 | 90.0 | 18.0 | 15 |
| Example 6 | 330 | 1 | 4.6 | 101 | 21.5 | 95.0 | 20.4 | 17 |
| Comparative Example 1 | 330 | 1 | 4.6 | 101 | 5.2 | 52.0 | 2.7 | 2.3 |
| Comparative Example 2 | 330 | 1 | 4.6 | 101 | 2.5 | 56.0 | 1.4 | 1.3 |

Example 8

A catalyst was prepared by using spherical high-purity active alumina (NST-7 supplied by Nikki-Universal Ltd.) having a size of 1.6 mm and properties described below. In this active alumina pores having a pore size of about 80 Å occupied about 80% by volume of the total pores.

Pore volume: 0.6 ml/g
Average pore size: 80 Å
Total BET specific surface area: 250 $m^2/g$
Pack density: 0.70 g/ml
Particle size: 1.6 mm
$Al_2O_3$ content: higher than 99.93% by weight
Na content: lower than 10 ppm
Si content: lower than 150 ppm
Fe content: lower than 100 ppm

Into 132 ml of pure water was thrown 287.5 g of chromium nitrate $Cr(NO_3)_2 \cdot 9H_2O$, and the mixture was heated at 70 to 80°C on a hot water bath to form a solution. The solution was cooled to room temperature and 400 g of the above-mentioned active alumina was immersed in the solution to make all of the catalyst solution adsorbed in the alumina. The alumina wetted with the catalyst solution was dried to the solid at 90°C on a hot water bath. The catalyst was further dried at 110°C for 2 hours in an air-circulating hot air drier.

An SUS vessel was charged with the dried catalyst and air was circulated at a space velocity [SV]o of 540 $hr^{-1}$, and the catalyst was calcined under a circulation of air at 200°C until the generation of heat in the catalyst layer stopped, and the temperature was elevated to 400°C and calcination was further conducted for 3 hours at this temperature. The calculated contents of $Cr_2O_3$ and $Al_2O_3$ were 12% by weight and 88% by weight, respectively. A reaction tube (formed of nickel) having an inner diameter of 25 mm and a height

of 1 m was packed with 100 ml of the calcined catalyst. Prior to the fluorination reaction of trichloroethylene CHCl = $CCl_2$ , the catalyst was subjected to the hydrogen fluoride treatment with hydrofluoric anhydride diluted with nitrogen and 100% hydrofluoric anhydride to activate the catalyst. The conditions for the treatment of the catalyst with hydrogen fluoride were as follows.

Fluoric anhydride concentration: 25 to 100 mole%

Temperature: 250 to 420°C

[SV]o: 400 $hr^{-1}$

Treatment time: about 15 hours

The gas phase fluorination reaction of trichloroethylene was carried out by using the thus-obtained catalyst.

The fluorination reaction of trichloroethylene was conducted under conditions of a reaction temperature of 350°C, a reaction pressure of 1 atmosphere, [SV]o of 123 $hr^{-1}$, an HF molar ratio of 3.4 to trichloroethylene, and HF and trichloroethylene flow rates of 0.425 mole/hr and 0.125 mole/hr, respectively. After the reaction became stationary, the formed gas was sampled and the reaction product was analyzed. The discharged gas was passed through an absorption column having two layers of an alkali and toluene to absorb unreacted HF and collect the product, and the gas was passed through an ethanol/dry ice trap to collect the unabsorbed product. The recovered toluene liquid containing the product was analyzed by gas chromatography.

As the result, it was found that the conversion of trichloroethylene was 88.4%, the yield of F-133a was 81.4% and the selectivity was 92.1%. The space time yield STY of F-133a was 121 (g/l of catalyst·hr).

Example 9

A catalyst was prepared by using high-purity active alumina having properties described below. In this active alumina, pores having a pore size of about 270 Å occupied about 80% by volume of the total pores.

Pore volume: 1.5 ml/g
Average pore size: 270 Å
Total BET specific surface area: 190 $m^2/g$
Pack density: 0.37 g/ml
Particle size: 3.2 mm
$Al_2O_3$ content: higher than 99.93% by weight
Na content: lower than 10 ppm
Si content: lower than 150 ppm
Fe content: lower than 100 ppm

The preparation of the catalyst was carried out in the same manner as described in Example 8, and the synthesis of F-134A was carried out while adjusting the conditions for the hydrogen fluoride treatment of the catalyst and the conditions for the fluorination reaction of F-133a in the same manner as in Example 8. The results are shown in Table 2.

Example 10

Using the same catalyst as used in Example 8, the fluorination reaction of trichloroethylene was carried out under the same conditions as described in Example 8 except that the reaction temperature was changed to 310°C. The results are shown in Table 2.

Example 11

A catalyst comprising 12% by weight of $Cr_2O_3$ and $Al_2O_3$ was prepared in the same manner as described in Example 8 except that 191.5 g of chromium chloride $CrCl_3 \cdot 6H_2O$ was used as the chromium starting material. The reaction tube was packed with 100 ml of the catalyst, and the hydrogen fluoride treatment of the catalyst and the fluorination reaction of trichloroethylene were carried out under the same conditions as described in Example 8. The results are shown in Table 2.

Example 12

The fluorination reaction of trichloroethylene was carried out in the same manner as described in Example 11 except that the reaction temperature was

changed to 400°C, the HF/trichloroethylene molar ratio was changed to 6.5 and the space velocity [SV]o was changed to 208 $hr^{-1}$. The results are shown in Table 2.

Example 13

A catalyst was prepared in the same manner as described in Example 8 except that 39.9 g of $CrCl_3 \cdot 6H_2O$ and 31.9 g of $(NH_4)_2CrO_4$ were used as the chromium starting material. The chromium content was 9.5% by weight. The hydrogen fluoride treatment of the catalyst and the fluorination reaction of trichloroethylene were carried out under the same conditions as described in Example 8. The results are shown in Table 2.

Example 14

The fluorination reaction of trichloroethylene was carried out in the same manner as described in Example 13 except that the space velocity [SV]o was changed to 170 $hr^{-1}$ and the HF/trichloroethylene molar ratio was changed to 5. The results are shown in Table 2.

Comparative Example 3

Using active alumina (KHA-24 supplied by Sumitomo Kagaku Kogyo Ltd.) having properties described below, a catalyst comprising 12% by weight of $Cr_2O_3$ and $Al_2O_3$ was prepared in the same manner as described in Example 8. This active alumina had only a broad pore size distribution within a pore size range of 40 to 500 Å, and the active alumina had about 20% of fine pores having a pore size smaller than 40 Å.

Pore volume: 0.53 ml/g
Total BET specific surface area: 150 $m^2$/g
Pack density: 0.72 g/ml
Particle size: 2 to 4 mm
$Al_2O_3$ content: higher than 98,4% by weight
Na content: lower than 1900 ppm
Si content: lower than 140 ppm
Fe content: lower than 210 ppm

The fluorination reaction of trichloroethylene was

carried out in the same manner as described in Example 8. The results are shown in Table 2.

Comparative Example 4

Using a commercially available chromium oxide/alumina catalyst (pellet having a diameter of 3 mm and a height of 3 mm; $Cr_2O_3$ content = 12% by weight), the hydrogen fluoride treatment and the fluorination reaction of trichloroethylene were carried out under the same conditions as described in Example 8. The results are shown in Table 2.

Table 2

| Example No. | Reaction Conditions: temperature (°C) | Reaction Conditions: pressure (atmosphere) | Reaction Conditions: HF/trichloroethylene molar ratio | Reaction Conditions: $[SV]_o$ ($hr^{-1}$) | Reaction Results: Conversion (%) of trichloroethylene | Reaction Results: Selectivity (%) of F-133a | Reaction Results: Yield (%) F-133a | Reaction Results: STY of F-133a (g/l of catalyst·hr) |
|---|---|---|---|---|---|---|---|---|
| Example 9 | 350 | 1 | 3.4 | 123 | 88.1 | 92.0 | 81.1 | 120 |
| Example 10 | 310 | 1 | 3.4 | 123 | 77.2 | 96.0 | 74.1 | 110 |
| Example 11 | 350 | 1 | 3.4 | 123 | 94.6 | 93.4 | 88.4 | 131 |
| Example 12 | 400 | 1 | 6.5 | 208 | 90.1 | 87.6 | 78.9 | 116 |
| Example 13 | 350 | 1 | 3.4 | 123 | 93.1 | 93.5 | 87.0 | 130 |
| Example 14 | 350 | 1 | 5.0 | 170 | 96.8 | 94.2 | 91.2 | 135 |
| Comparative Example 3 | 350 | 1 | 3.4 | 123 | 60.1 | 85.0 | 51.1 | 76 |
| Comparative Example 4 | 350 | 1 | 3.4 | 123 | 62.4 | 88.5 | 55.2 | 82 |

Example 15

A catalyst was prepared by using spherical high-purity active alumina (NST-3 supplied by Nikki-Universal) having a size of 3.2 mm and properties described below. This active alumina was one prepared by the sol/gel oil-dropping method, and pores having a pore size of about 1270 Å occupied about 80% by volume of the total pores.

Pore volume: 1.5 ml/g
Average pore size: 270 Å
Total BET specific surface area: 190 $m^2/g$
Pack density: 0.37 g/ml
Particle size: 3.2 mm
$Al_2O_3$ content: higher than 99.93% by weight
Na content: lower than 10 ppm
Si content: lower than 150 ppm
Fe content: lower than 100 ppm

Into 370 ml of deionized water was thrown 365 g of nickel chloride $NiCl_2 \cdot 6H_2O$, and the mixture was heated at 70 to 80°C on a hot water bath to form a solution. The solution was cooled to room temperature and 369 g of the above-mentioned active alumina was immersed in the solution to make all of the catalyst solution adsorbed in the alumina. The alumina wetted with the catalyst solution was dried to the solid at 90°C on a hot water bath. The catalyst was further dried at 120°C for 3 hours in an air-circulating hot air drier.

An SUS vessel was charged with the dried catalyst and air was circulated at a space velocity [SV]o of 540 $hr^{-1}$, and the catalyst was calcined under circulation of air at 200°C until the generation of heat in the catalyst layer stopped, and the temperature was elevated to 400°C and calcination was further conducted for 3 hours at this temperature. The contents of NiO and $Al_2O_3$ were 23.7% by weight and 76.3% by weight, respectively. A reaction tube (formed of nickel) having an inner diameter of 25 mm and a height of 1 m was

packed with 150 ml of the calcined catalyst. Prior to the fluorination treatment of F-133a, the catalyst was subjected to partial fluorination with hydrofluoric anhydride diluted with nitrogen and 100% hydrofluoric anhydride to activate the catalyst. The conditions for the treatment of the catalyst with hydrofluoric anhydride were as follows.

Hydrofluoric anhydride concentration: 25 to 100 mole%

Temperature: 250 to 420°C

[SV]o: 400 $hr^{-1}$

Treatment time: about 15 hours

The gas phase fluorination reaction of F-133a was carried out by using the thus-obtained catalyst. The formed gas coming form the reactor was passed through an aqueous solution of an alkali to effect absorption of unreacted hydrofluoric anhydride and formed HCl, and dehydration was carried out in a molecular sieve 3A column and metering was performed by a gas meter. The formed gas was analyzed by the gas chromatography. The analysis was carried out when the reaction became stationary after the passage of hydrofluoric anhydride and F-133a through the catalyst.

The fluorination reaction of F-133a was conducted under conditions of a reaction temperature of 330°C, a reaction pressure of 1 atmosphere, a hydrofluoric anhydride molar ratio (HF molar ratio) of 4.0 to F-133a and a space velocity [SV]o of 90 $hr^{-1}$. The conversion of F-133a was 13.1%, and the yield and selectivity of 1,2,2,2-tetrafluoroethane ($CH_2F-CF_3$ , F-134a) were 12.0% and 91.6%, respectively. The space time yield STY of F-134a was 9.8 (g/l of catalyst•hr).

<u>Example 16</u>

A catalyst was prepared by using high-purity active alumina having properties described below. In this active alumina, pores having a pore size of about 80 Å occupied about 80% by volume of the total pores.

Pore volume: 0.6 ml/g

Average pore size: 80 Å

Total BET specific surface area: 250 $m^2/g$

Pack density: 0.70 g/ml

Particle size: 1.6 mm

$Al_2O_3$ content: higher than 99.93% by weight

Na content: lower than 10 ppm

Si content: lower than 150 ppm

Fe content: lower than 100 ppm

The preparation of the catalyst was carried out in the same manner as described in Example 15, and in the formed catalyst, the contents of NiO and $Al_2O_3$ were 23.7% by weight and 76.3% by weight, respectively. The synthesis of F-134A was carried out while adjusting the amount packed of the catalyst, the conditions for the hydrogen fluoride treatment of the catalyst and the conditions for the fluorination reaction of F-133a in the same manner as in Example 15. The results are shown in Table 3.

Example 17 through 19

Using the same catalyst as used in Example 15, the fluorination reaction of F-133a was carried out in the same manner as described in Example 15 except that the reaction conditions were changed. The results and the reaction conditions are shown in Table 3.

Example 20

A catalyst comprising 23.7% by weight of NiO and $Al_2O_3$ was prepared in the same manner as described in Example 15 except that 446 g of $Ni(NO_3)_2 \cdot 6H_2O$ was used as the nickel starting material. The reaction tube was packed with 100 ml of the catalyst, and the hydrogen fluoride treatment of the catalyst and the fluorination reaction of F-133a were carried out under the same conditions as described in Example 15. The results are shown in Table 3.

Comparative Example 5

Using active alumina (KHA-24 supplied by Sumitomo

Kagaku Kogyo) having properties described below, a catalyst comprising 23.7% by weight of NiO and $Al_2O_3$ was prepared in the same manner as described in Example 15. This active alumina had only a broad pore size distribution within a pore size range of 40 to 500 Å, and the active alumina had about 20% of fine pores having a pore size smaller than 40 Å.

Pore volume: 0.53 ml/g

Total BET specific surface area: 150 $m^2/g$

Pack density: 0.72 g/ml

Particle size: 2 to 4 mm

$Al_2O_3$ content: higher than 98.4% by weight

Na content: lower than 1900 ppm

Si content: lower than 140 ppm

Fe content: lower than 210 ppm

A reaction tube was packed with 100 ml of the catalyst, and the hydrogen fluoride treatment and the fluorination of F-133a were carried out under the same conditions as described in Example 15. The results are shown in Table 3.

Comparative Example 6

Using a commercially available nickel oxide/alumina catalyst (pellet having a diameter of 5 mm and a height of 5 mm; NiO content = 20% by weight), the hydrogen fluoride treatment of the catalyst and the fluorination reaction of F-133a were carried out under the same conditions as described in Example 15. The results are shown in Table 3.

Table 3

| Example No. | Reaction Conditions: reaction temperature (°C) | reaction pressure (atmosphere) | HF/F-133a molar ratio | [SV]o ($hr^{-1}$) | Reaction Results: Conversion (%) of F-133a | Selectivity (%) of F-134a | Yield (%) of F-134a | STY of F-134a (g/l of catalyst•hr) |
|---|---|---|---|---|---|---|---|---|
| Example 16 | 330 | 1 | 4.0 | 90 | 15.2 | 92.8 | 14.1 | 11.6 |
| Example 17 | 330 | 1 | 8.4 | 90 | 23.2 | 95.3 | 22.1 | 10.1 |
| Example 18 | 330 | 1 | 4.0 | 235 | 13.2 | 92.0 | 12.1 | 25.9 |
| Example 19 | 390 | 1 | 8.4 | 430 | 26.5 | 91.0 | 24.1 | 50.2 |
| Example 20 | 330 | 1 | 4.0 | 90 | 15.7 | 90.0 | 14.1 | 11.6 |
| Comparative Example 5 | 330 | 1 | 4.0 | 90 | 5.2 | 52.0 | 2.7 | 2.2 |
| Comparative Example 6 | 330 | 1 | 4.0 | 90 | 2.4 | 56.0 | 1.35 | 1.1 |

Example 21

The fluorination reaction of F-133a containing 2% of 1-chloro-2,2-difluoroethylene ($CHCl = CF_2$) was carried out in the same manner as described in Example 15 except that the HF molar ratio to 1-chloro-2,2-difluoroethylene was changed to 2, the space velocity [SV]o was changed to 500 $hr^{-1}$ and the temperature was changed to 200°C.

1-Chloro-2,2-difluoroethylene was not detected in the product, but it was confirmed that F-133a remained unreacted.

It is seen that, at a low temperature as described above, only an addition of hydrogen fluoride to the unsaturated double bond occurs but a substitution of chlorine with fluorine does not occur.

Example 22

A catalyst containing Ag, Fe, Co, Mn or Cu was prepared by using the same alumina carrier as used in Example 15. The amount deposited of the metal per gram of the carrier was the same as the amount deposited of Ni in Example 15, that is, 4.2 g mg-atom metal/g-$Al_2O_3$. Nitrates of Ag and Fe and chlorides of Co, Mn and Cu were used as the starting salt. The preparation of the catalyst and the hydrogen fluoride treatment of the catalyst were carried out in the same manner as described in Example 15.

With respect to each of the catalysts, using the same reactor packed with the same amount (150 ml) of the catalyst as in Example 15, the fluorination reaction of F-133a was carried out while conducting the hydrogen fluoride treatment of the catalyst and the analysis of the product under the same conditions as described in Example 15. The fluorination was conducted under conditions of a temperature of 390°C, an HF molar ratio of 8 or 8.4 to F-133a and a space velocity [SV]o of 430 $hr^{-1}$. The results are shown in Table 4.

Table 4

| Metal | Ag | Fe | Co | Mn | Cu |
|---|---|---|---|---|---|
| HF/F-133a (Starting material molar ratio) | 8.4 | 8.4 | 8.0 | 8.0 | 8.0 |
| Yield (%) of F-134a | 15.1 | 11.2 | 8.1 | 4.5 | 3.6 |
| Selectivity (%) of F-134a | 95.0 | 75.1 | 64.0 | 60.1 | 55.0 |

Example 23

Using the same catalyst as used in Example 15, the synthesis of tetrafluoromethane ($CF_4$ , F-14), chlorodifluoromethane ($CHClF_2$ , F-22) and F-133a were carried out. The hydrogen fluoride treatment of the catalyst was carried out in the same manner as described in Example 15. The reaction results together with the reaction conditions are shown in Table 5.

Table 5

| Starting Material<br>Reaction Product | $CCl_2F_2$<br>F-14 | | $CHCl_3$<br>F-22 | | $CHCl = CCl_2$<br>F-133a | |
|---|---|---|---|---|---|---|
| Reaction Conditions | | | | | | |
| temperature (°C) | 270 | 300 | 200 | 175 | 350 | 310 |
| $[SV]o[hr^{-1}]$ | 135 | 180 | 670 | 670 | 124 | 124 |
| HF ratio [-] | 0.8 | 0.6 | 1.0 | 1.0 | 3.4 | 3.4 |
| Reaction Results | | | | | | |
| yield [%] | 25 | 40.4 | 49.5 | 12.6 | 91.5 | 76.1 |
| selectivity [%] | 99 | 99 | 49.5 | 16.0 | 94.5 | 98.5 |
| STY [g/l of catalyst•hr] | 74 | 180 | 640 | 163 | 136 | 113 |

Example 24

Using the catalyst of Example 15 and the Ag catalyst of Example 22, the fluorination reaction of 1,1,1-trichloroethane ($CCl_3$-$CH_3$) was carried out. The hydrogen fluoride treatment of the catalysts and the fluorination reaction were carried out in the same manner as described in Example 15. The reaction results together with the reaction conditions are shown in Table 6.

Table 6

| Metal | | Ni | | | Ag |
|---|---|---|---|---|---|
| Reaction Conditions | temperature [°C] | 200 | 250 | 350 | 200 |
| | $[SV]_o[hr^{-1}]$ | 108 | 124 | 124 | 108 |
| | HF molar ratio [-] | 1.3 | 3 | 3 | 1.3 |
| Reaction Results | yield [%] 141b*1 | 74.4 | 0 | 0 | 30.0 |
| | 142b*2 | 12.9 | 0 | 0 | 5.2 |
| | 143a*3 | 7.9 | 95 | 92 | 3.2 |
| | STY of 141b [g/l of catalyst·hr] | 183 | - | - | 73.5 |

Note

*1: 1-fluoro-1,1-dichloroethane ($CCl_2$-$CH_3$)
*2: 1-chloro-1,1-difluoroethane ($CClF_2$-$CH_3$)
*3: 1,1,1-trifluoroethane ($CF_3$-$CH_3$)

Example 25

A chromia-alumina catalyst comprising 24% by weight of $Cr_2O_3$ and 76% by weight of $Al_2O_3$ was prepared in the same manner as described in Example 15 by using chromium chloride $CrCl_3 \cdot 6H_2O$ as the starting chromium material and the same alumina as used in Example 15 as the carrier, and by using this catalyst, the synthesis of F-14, F-22 and hexafluoroethane ($CF_3$-$CF_3$ , F-116) was carried out. The hydrogen fluoride treatment of the catalyst and the fluorination reaction were carried out in the same manner as described in Example 15. The reaction results together with the reaction conditions are shown in Table 7.

Example 26

The synthesis of F-14 was carried out in the same manner as described in Example 15 by using the Fe catalyst used in Example 22. The reaction results

together with the reaction conditions are shown in Table 7.

Table 7

| Example No.<br>Starting Material<br>Reaction Product | $CCl_2F_2$<br>F-14 | | 25<br>$CHCl_3$<br>F-22 | | $CF_3$-$CF_2Cl$<br>F-116 | | 26<br>$CCl_2F_2$<br>F-14 |
|---|---|---|---|---|---|---|---|
| Reaction Conditions | | | | | | | |
| temperature (°C) | 274 | 301 | 200 | 175 | 455 | 395 | 274 |
| $[SV]o[hr^{-1}]$ | 135 | 181 | 670 | 670 | 100 | 100 | 135 |
| HF ratio [-] | 0.79 | 0.58 | 1.0 | 1.0 | 1.0 | 3.9 | 0.79 |
| Reaction Results | | | | | | | |
| yield [%] | 30.8 | 45.4 | 56 | 16 | 32.8 | 5.8 | 10.1 |
| selectivity [%] | 99 | 99 | 56 | 18 | 95.0 | 80.6 | 98 |
| STY [g/l of catalyst•hr] | 91 | 205 | 720 | 210 | 101 | 7 | 30 |

Example 27

The fluorination reaction of perchloroethylene was carried out using the same chromia-alumina catalyst as used in Example 25. The hydrogen fluoride treatment of the catalyst and the fluorination reaction were carried out in the same manner as described in Example 25. The reaction conditions and the reaction results were as shown below.

Reaction Conditions

Temperature: 250°C

[SV]o: 240 $hr^{-1}$

HF molar ratio: 3.7

Reaction results

Yield: 20.0% for 1,1-dichloro-2,2,2-trifluoroethane ($CHCl_2-CF_3$ , F-123), 6.4% for 1-chloro-1,2,2,2-tetrafluoroethane ($CHClF-CF_3$ , F-124), 4.1% for $CCl_2 = CClF + CHCl_2-CClF_2$ , STY of F-123 = 70 (g/l of catalyst•hr)

Example 28

The fluorination reaction of 1,1,1-trichloroethane ($CCl_3-CH_3$) was carried out using the same chromia-alumina catalyst as used in Example 25. The hydrogen fluoride treatment of the catalyst and the fluorination reaction were carried out in the same manner as described in Example 27. The reaction conditions and the obtained results were as shown below.

Reaction Conditions

Temperature: 200°C

[SV]o: 108 $hr^{-1}$

HF molar ratio: 1.3

Reaction Results

Yield: 80% for 141b, 10% for 142b, 5% for 143a, STY of 141b = 196 g/l of catalyst•hr

Example 29

Using the same Co catalyst as used in Example 22,

the fluorination reaction of 1,1,1-trichloroethane was carried out in the same manner as described in Example 25 under conditions of a temperature of 200°C, a space velocity of [SV]o of 108 $hr^{-1}$, and an HF molar ratio of 1.3. The yield of 141b was 16%.

Example 30

Using the same chromia-alumina catalyst as used in Example 25, the fluorination reaction of trifluorochloroethylene ($CClF=CF_2$ , F-1113) was carried out. The hydrogen fluoride treatment of the catalyst and the fluorination reaction were conducted in the same manner as described in Example 25. The reaction conditions and reaction results were as shown below.

Reaction Conditions

Temperature: 300 or 350°C

[SV]o: 210 $hr^{-1}$

HF molar ratio: 1.65

Reaction Results

| Temperature [°C] | Yield [%] F-124 | F-125 | Selectivity F-124 | F-125 | STY [g/l of catalyst·hr] |
|---|---|---|---|---|---|
| 300 | 66.8 | 2.8 | 86.0 | 3.6 | 330 |
| 350 | 10.1 | 68.1 | 10.1 | 68.1 | 290 |

Note

F-124: $CHClF-CF_3$ , 1,1,1,2-tetrafluorochloroethane

F-125: $CHF_2-CF_3$: 1,1,1,2,2-pentafluoroethane

Example 31

The fluorination reaction of benzotrichloride was carried out using the catalyst prepared in Example 15. The reaction product was cooled, collected and fractionated to obtain a product. The synthesis was carried out under conditions of a reaction temperature of 300°C, a space velocity [SV]o of 90 $hr^{-1}$ and a hydrofluoric acid molar ratio of 9.5 to

benzotrichloride. The yield of benzotrifluoride was 74%.

Example 32

The fluorination reaction of hexachloroacetone was carried out using the catalyst prepared in Example 15. The gas formed by the reaction was cooled and liquefied by passing the gas through a sodium fluoride layer and was then subjected to fractionation to obtain a product. The synthesis was conducted under reaction conditions of a reaction temperature of 350°C, a space velocity [SV]o of 600 $hr^{-1}$ and a hydrogen fluoride molar ratio of 18 to hexachloroacetone. Hexafluoroacetone, pentafluoromonochloroacetone and tetrafluorodichloroacetone were obtained in yields of 14, 22 and 26%, respectively.

Industrial Applicability

The present invention can be advantageously applied to the preparation of an organic fluorine compound valuable as a cooling medium, an aerosol propellant, a foaming agent, a detergent and the like.

## CLAIMS

1. A process for the preparation of an organic fluorine compound, which comprises reacting an organic chlorine compound or an organic unsaturated compound with hydrogen fluoride in the presence of a catalyst comprising a catalyst carrier composed of active alumina in which pores having an average pore size of 40 to 500 Å occupy 70 to 90% of the whole pores, and at least one fluoride of a metal selected from the group consisting of nickel, cobalt, iron, manganese, chromium, copper and silver, which is supported on the catalyst carrier.

2. A process according to claim 1, wherein the metal fluoride is a partial fluoride of the metal obtained by supporting a compound of the metal on the catalyst carrier and subjecting the metal compound-supported alumina carrier to a gas phase hydrogen fluoride treatment.

3. A process according to claim 1 or 2, wherein a solution of an inorganic or organic salt of the metal is supported on the alumina carrier, the alumina carrier is heated at 200 to 500°C in oxygen or an oxygen-containing gas, and then, the hydrogen fluoride treatment is carried out at 200 to 450°C.

4. A process according to claim 1, wherein the active alumina has a purity of at least 99.9% by weight and a sodium content lower than 100 ppm.

5. A process according to claim 1 or 4, wherein in the active alumina, the volume of pores having a size of at least 40 Å is 0.55 to 1.6 ml/g.

6. A process according to claim 1, wherein trichloroethylene is used as the organic unsaturated organic compound and is reacted with hydrogen fluoride at a temperature of 200 to 450°C and a hydrogen fluoride molar ratio of 3 to 10 to trichloroethylene to form 1-chloro-2,2,2-trifluoroethane.

7. A process according to claim 1, wherein

1-chloro-2,2,2-trifluoroethane is used as the organic chlorine compound and is reacted with hydrogen fluoride at a temperature of 300 to 420°C and a hydrogen fluoride molar ratio of 1 to 10 to 1-chloro-2,2,2-trifluoroethane to form 1,2,2,2-tetrafluoroethane.

8. A process according to claim 1, wherein perchloroethylene is used as the organic unsaturated compound and is reacted with hydrogen fluoride at a temperature of 200 to 400°C and a hydrogen fluoride molar ratio of 3 to 6 to perchloroethylene to form 1,1-dichloro-2,2,2-trifluoroethane.

9. A process according to claim 1, wherein 1,1,1-trichloroethane is used as the organic chlorine compound and is reacted with hydrogen fluoride at a temperature of 150 to 400°C and a hydrogen fluoride molar ratio of 1 to 4 to 1,1,1-trichloroethane to form 1,1,1-dichlorofluoroethane and/or 1,1,1-difluorochloroethane.

10. A process according to claim 1, wherein chloroform is used as the organic chlorine compound and is reacted with hydrogen fluoride at a temperature of 150 to 350°C and a hydrogen fluoride molar ratio of 0.5 to 3 to chloroform to form difluoromethane.

11. A process according to claim 1, wherein difluorodichloromethane and/or trifluorochloromethane is used as the organic chlorine compound and is reacted with hydrogen fluoride at a temperature of 200 to 460°C and a hydrogen fluoride molar ratio of 0.5 to 3 to difluorodichloromethane and/or trifluorochloromethane to form tetrafluoromethane.

12. A process according to claim 1, wherein trifluorochloroethylene is used as the organic unsaturated compound and is reacted with hydrogen fluoride at a temperature of 200 to 450°C and a hydrogen fluoride molar ratio of 1 to 5 to trifluorochloroethylene to form 1,1,1,2-tetrafluorochloroethane and/or 1,1,1,2,2-pentafluoroethane.

# INTERNATIONAL SEARCH REPORT

International Application No PCT/JP89/00020

**I. CLASSIFICATION OF SUBJECT MATTER** (if several classification symbols apply, indicate all) [6]

According to International Patent Classification (IPC) or to both National Classification and IPC

$Int.Cl^4$ C07B39/00, C07C17/08, 17/20, 19/08

**II. FIELDS SEARCHED**

Minimum Documentation Searched [7]

| Classification System | Classification Symbols |
|---|---|
| IPC | C07B39/00, C07C17/08, 17/20, 19/08 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, A, 59-108726 (Central Glass Co., Ltd.) 23 June 1984 (23. 06. 84) (Family: none) | 1-12 |
| Y | JP, A, 59-80332 (General Electric Co.) 9 May 1984 (09. 05. 84) & US, A, 4465786 | 1-12 |
| Y | JP, A, 53-18503 (Asahi Chemical Industry Co., Ltd.) 20 February 1978 (20. 02. 78) (Family: none) | 1-12 |
| Y | JP, Bl, 55-12297 (Montecatini Edison S.p.A.) 1 April 1980 (01. 04. 80) & US, A, 3787331 | 1-12 |
| Y | JP, Bl. 52-33086 (Dynamit Nobel A.G.) 26 August 1977 (26. 08. 77) & DE, A, 2000200 | 1-12 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 22, 1989 (22. 03. 89) | April 3, 1989 (03. 04. 89) |
| **International Searching Authority** | **Signature of Authorized Officer** |
| Japanese Patent Office | |

International Application No. PCT/JP89/00020

**FURTHER INFORMATION CONTINUED FROM THE SECOND SHEET**

| | | |
|---|---|---|
| Y | JP, B1, 41-203 (Imperial Chemical Industries P.L.C.) 11 January 1966 (11. 01. 66) | 1-12 |
| Y | JP, B1, 39-21984 (Union Carbide Corp.) 6 October 1964 (06. 10. 64) | 1-12 |
| Y | JP, B1, 39-17263 (Union Carbide Corp.) 20 August 1964 (20. 08. 64) | 1-12 |
| Y | JP, B1, 39-10310 (Union Carbide Corp.) 11 June 1964 (11. 06. 64) | 1-12 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers .... .. , because they relate to subject matter not required to be searched by this Authority, namely:

2.☐ Claim numbers ......... , because they relate to parts of the international application that do not comply with the prescribed requirements to such an extent that no meaningful international search can be carried out, specifically:

3.☐ Claim numbers ..... ....., because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

International Application No. PCT/JP89/00020

FURTHER INFORMATION CONTINUED FROM THE SECOND SHEET

| | | |
|---|---|---|
| Y | JP, B1, 37-16583 (Showa DEnko Kabushiki Kaisha) 16 October 1962 (16. 10. 62) | 1-12 |

V.☐ OBSERVATIONS WHERE CERTAIN CLAIMS WERE FOUND UNSEARCHABLE [1]

This international search report has not been established in respect of certain claims under Article 17(2) (a) for the following reasons:

1.☐ Claim numbers ..... ....., because they relate to subject matter not required to be searched by this Authority, namely:

2.☐ Claim numbers ..........., because they relate to parts of the international application that do not comply with the prescribed requirements to such an extent that no meaningful international search can be carried out, specifically:

3.☐ Claim numbers ............, because they are dependent claims and are not drafted in accordance with the second and third sentences of PCT Rule 6.4(a).

VI.☐ OBSERVATIONS WHERE UNITY OF INVENTION IS LACKING [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.